# EUROPEAN PATENT SPECIFICATION

(11) **EP 3 823 655 B1**
(45) Date of publication and mention of the grant of the patent: **29.11.2023**
(21) Application number: 19742020.1
(22) Date of filing: 16.07.2019
(51) Int. Cl.: A61K 36/9066, A61K 9/00, A61P 15/02, A61K 36/61, A61K 36/28, A61K 47/44

(54) **COMPOSITIONS FOR THE TREATMENT OF VULVODYNIA**
ZUSAMMENSETZUNGEN ZUR BEHANDLUNG VON VULVODYNIA
COMPOSITIONS POUR LE TRAITEMENT DE LA VULVODYNIE

(30) Priority: 17.07.2018 EP 18382530
(43) Date of publication of application: 26.05.2021
(73) Proprietor: Procare Health Iberia, S.L., 46450 Benifaió (ES)
(72) Inventor: TOMASI SCIANÒ, Giuseppe Giovanni, 20730 Azpeitia, Gipuzkoa (ES); LARRAÑAGA LAZKANO, Lidia, 20730 Azpeitia, Gipuzkoa (ES); MOLERO RODRÍGUEZ, Francisca, 08921 Santa Coloma de Gramenet, Barcelona (ES)
(74) Representative: ZBM Patents - Zea, Barlocci & Markvardsen
(86) International application number: PCT/EP2019/069078
(87) International publication number: WO 2020/016210

(56) References cited:
- EP-A2- 2 014 295
- WO-A1-2011/044122
- WO-A1-2015/000064
- US-A1- 2010 233 322
- DATABASE GNPD [Online] MINTEL; 5 August 2015 (2015-08-05), anonymous: "Natural Vita Sun Stick SPF 50+ PA++", XP055539368, retrieved from www.gnpd.com Database accession no. 3328805
- DATABASE WPI Week 201358 Thomson Scientific, London, GB; AN 2013-L90876 XP055539981, & RU 2 486 911 C1 (SUPRUN A E) 10 July 2013 (2013-07-10)
- MURINA FILIPPO ET AL: "Thymol, eugenol and lactobacilli in a medical device for the treatment of bacterial vaginosis and vulvovaginal candidiasis.", THE NEW MICROBIOLOGICA 07 2018, vol. 41, no. 3, July 2018 (2018-07), pages 220-224, XP055539547, ISSN: 1121-7138
- MIRANDA VARELLA PEREIRA G ET AL: "A systematic review of drug treatment of vulvodynia: evidence of a strong placebo effect.", BJOG : AN INTERNATIONAL JOURNAL OF OBSTETRICS AND GYNAECOLOGY SEP 2018, vol. 125, no. 10, September 2018 (2018-09) , pages 1216-1224, XP055539395, ISSN: 1471-0528

## Description

### Field of the Invention

The present invention relates to combinations comprising *Calendula officinalis* flower oil, *Curcuma longa* root extract, and *Eugenia caryophyllus* bud extract, and to their use in the treatment and/or prevention of vulvodynia. The invention also discloses the preparation of said combinations as well as pharmaceutical compositions comprising them.

### Background of the Invention

Vulvodynia is a chronic vulvar pain syndrome affecting as many as 28% of women within their lifetime. Recognised in 1987 as vulvar vestibulitis, vulvodynia is now defined as persistent vulvar pain which is not secondary to any other obvious pathological state, such as active microbial infection or dermatological conditions. The exact cause of vulvodynia is unknown and is at present believed to involve a number of factors such as genetics, immunology, and even diet. In line with these circumstances, vulvodynia is generally diagnosed by ruling out other possible causes for the symptoms suffered by the patient.

Vulvodynia can be either localized, when it affects at least a portion of the vulvar vestibule or the clitoris; or generalised, when it affects the vulva as a whole. The pain, which is often described as cutting, burning or stinging, usually involves rawness, irritation, or itching, and can be provoked by touch (e.g. during tampon insertion or sexual intercourse), can be unprovoked, or both.

Current lines of treatment of vulvodynia include one or more of topical therapy, oral medication, parenteral medication, surgery, physical therapy, psychological therapy, or dietary regimes.

As regards topical therapy, treatment strategies are guided mainly by expert opinion, rather than an evidence-based approach from randomised clinical trials. As a general rule, recourse to medication is increased as symptoms fail to remit or worsen. The therapy thus usually involves a first line of treatment not involving or minimally involving drugs, involving for instance simply minimising environmental irritants to the vulva, e.g. by reducing or eliminating detergent use, wearing exclusively cotton underwear, or refraining from wearing tight clothing. If the pain persists, these first line measures are often followed by or combined with the use of topical drugs to relieve pain, such as anaesthetics, or also muscle relaxants, antidepressants or anticonvulsants.

A constant need exists to enrich the number of treatments available to patients suffering from vulvodynia. In particular, there is a need for treatments which may complement first line treatment of vulvodynia and are not aggressive from the therapeutic point of view.

### Summary of the Invention

The present inventors have now surprisingly found a combination of natural substances which is useful in the treatment of vulvodynia. Specifically, a combination comprising *Calendula officinalis* flower oil, *Curcuma longa* root extract and *Eugenia caryophyllus* bud extract has been shown *in vivo* to be able to reverse and control persistent vulvar pain, without alternations in the vulva and vagina.

The invention is set out in the appended set of claims and additionally also defined hereinafter in the detailed description.

### Detailed Description of the Invention

The first aspect of the present invention is directed to a combination comprising:
- *Calendula officinalis* flower oil;
- *Curcuma longa* root extract; and
- *Eugenia caryophyllus* bud extract,
each present in an amount of 1-3% by weight with respect to the total weight of the combination.

***Calendula officinalis*** is a plant belonging to the *Asteraceae* family. Flowers of the plant have long been used as a medicinal herb, and are also employed as a dye for fabrics, foods or cosmetics.

The mixture of components extracted from Calendula officinalis flower can comprise essential oils such as oxygenated mono and sesquiterpenes, e.g. carvone, geranylacetone, caryophyllene ketone, menthone, isomenthone, γ-terpinene, δ-cadinene, cariophyllene, α- and β-ionones, 5,6-epoxy-β-ionone, pedunculatine, dihydroactinidiolide, or α-muurolene; tritepene alcohols such as arnidiol, faradiol, α- and β-amyrin, faradiol-3-myristic acid, lupol, taraxasterol, faradiol-3-palmytic acid, or calenduladiol; or carotenoids such as celenduline, α-, β- and γ-carotene, lycopene, rubixanthin, violaxanthin, citroxanthin, zein, chrysanthemumxanthin, flavoxanthin, auroxanthin, lutein, microxanthin, or 5,6-epoxycarotene.

The Calendula officinalis flower oil can be obtained by extracting the flowers or a powder thereof with an organic solvent or an oil. In order to prepare the extract, the flowers can be treated, in particular they can be boiled, cured, dried and/or brought to powder form before carrying out the extraction.

In an embodiment, the Calendula officinalis flower oil is obtained by extraction of the flowers of the plant with an organic solvent. Examples of suitable organic solvents are aromatic hydrocarbons, such as toluene or xylene; aliphatic hydrocarbons such as diethyl ether, heptane, pentane, hexane, cyclohexane; chlorinated hydrocarbons such as ethylene dichloride, dichloromethane, trichlorethylene; ketones such as acetone; esters such as ethyl acetate; alcohols such as methanol, ethanol, propanol or butanol, or glycerol or propylene glycol; or any mixture thereof. Preferably, the organic solvent is dichloromethane or hexane. After extraction, solids in the extraction mixture may be removed such as by filtration, and the solvent evaporated to yield the Calendula officinalis flower oil.

In another embodiment, the extraction is performed by adding at least one oil to the flowers, more specifically by macerating the flowers with the oil. The oil employed for the extraction is preferably an oil of natural origin. Preferably, the oil of natural origin is selected from soybean oil, sunflower oil, almond oil, safflower oil, corn oil, sesame oil, rapeseed oil, linseed oil, cottonseed oil, rice bran oil, perilla oil, castor oil, olive oil, evening primrose oil, tsubaki oil, coconut oil, palm oil, hemp seed oil, tung oil, kapok oil and tea seed oil. Preferably, the oil is a pharmaceutically acceptable oil. More preferably, the oil of natural origin is sunflower oil. After extraction, solids in the extraction mixture may be removed such as by filtration.

In an embodiment, when an oil is employed for extracting the flowers, the final oil (the oil with the extracted components) comprises between about 0.5 and about 20%, preferably between about 0.5 and about 10%, more preferably between about 1 and about 5% by weight components extracted from the flowers with respect to the total weight of the final oil. The final oil may additionally comprise added components such as at least one antioxidant selected from those described further below, which can be present in amounts of about 0.001 % to about 3%, preferably about 0.01 to about 0.1 %, with respect to the total weight of the final oil. The remaining weight of the final oil (up to 100% weight) is the oil employed for the extraction.

In another embodiment, the Calendula officinalis flower oil is obtained by extraction of the flowers of the plant using supercritical carbon dioxide as the solvent. For instance, such an extraction may be carried out with carbon dioxide at a temperature of carbon dioxide between about 31°C and about 70°C, preferably between about 24 and about 55°C, and at a pressure of carbon dioxide comprised between about 74 and about 300 bar, preferably between about 100 and about 300 bar.

The Calendula flower oil can also be obtained from different commercial sources, such as from Parchem fine & specialty chemicals ("Marigold pot extract"; CAS no. 84776-23-8), from Croda Inc. (as Calendula Oil Monarom^{®}), or from Provital S.A. (as 1210000G - Aceite de Caléndula).

***Curcuma longa*** is a rhizomatous herbaceous perennial plant of the ginger family, Zingiberaceae. It is used extensively in Indian and Asian cuisine, and is also employed as a coloring and flavoring agent, or as a botanical supplement. Generally, the terms "turmeric" and "Curcuma longa" are used interchangeably; the same applies to the terms "root" and "rhizome", which include the primary rhizomes and the various multiple cylindrical secondary rhizomes that grow downward from the primary rhizomes.

The mixture of components extracted from *Curcuma longa* root can comprise the curcuminoid compounds such as curcumin, desmetoxycucumin, and bisdesmethoxyurcumin; essential oils such as sesquiterpenes, α-phellandrene, cineole, sabinene and borneol; or carbohydrates such as starch.

The *Curcuma longa* root extract can be obtained by extracting the root or a powder thereof with an organic solvent or an oil. In order to prepare the extract, the root can be treated, in particular it can be boiled, cured, dried and/or brought to powder form before carrying out the extraction.

In an embodiment, the extraction is performed by adding an organic solvent to the curcuminoid-containing material (i.e. to the treated or untreated root). Examples of suitable solvents are aromatic hydrocarbons, such as toluene or xylene; aliphatic hydrocarbons such as diethyl ether, heptane, pentane, hexane, cyclohexane; chlorinated hydrocarbons such as ethylene dichloride, dichloromethane, trichlorethylene; ketones such as acetone; esters such as ethyl acetate; alcohols such as methanol, ethanol, propanol or butanol, or glycerol or propylene glycol; or any mixture thereof. The organic solvent is preferably a solvent that is pharmaceutically acceptable, such as acetone, methanol, ethanol, propanol or butanol, glycerol, ethyl acetate, diethyl ether, hexane, cyclohexane or propylene glycol. After extraction, solids in the extraction mixture may be removed such as by filtration, and the solvent is optionally evaporated.

In another embodiment, the extraction is performed by adding at least one oil to the curcuminoid-containing material, more specifically by macerating the curcuminoid-containing material with the oil. Preferably, the oil is an oil of natural origin. In a preferred embodiment, the oil is selected from soybean oil, sunflower oil, almond oil, safflower oil, corn oil, sesame oil, rapeseed oil, linseed oil, cottonseed oil, rice bran oil, perilla oil, castor oil, olive oil, evening primrose oil, tsubaki oil, coconut oil, palm oil, hemp seed oil, tung oil, kapok oil and tea seed oil. Preferably, the oil is a pharmaceutically acceptable oil. More preferably, the oil is sunflower oil. After extraction, solids in the extraction mixture may be removed such as by filtration.

In an embodiment, when an oil is employed for extracting the curcuminoid-containing material, the final oil (the oil with the extracted components) comprises between about 20 and about 80%, preferably between about 20 and about 60%, more preferably between about 30 and about 50%, even more preferably about 40% by weight components extracted from the curcuminoid-containing material with respect to the total weight of the final oil. The final oil may additionally comprise added components such as at least one antioxidant selected from those described further below, which can be present in amounts of about 0.001% to about 3%, preferably about 0.1 to about 0.5%, more preferably about 0.3%, with respect to the total weight of the final oil. The remaining weight of the final oil (up to 100% weight) is the oil employed for the extraction.

In another embodiment the extraction is an extraction using supercritical carbon dioxide as the solvent. For instance, such an extraction may be carried out with carbon dioxide at a temperature of carbon dioxide between about 31°C and about 70°C, preferably between about 24 and about 55°C, and at a pressure of carbon dioxide comprised between about 74 and about 300 bar, preferably between about 100 and about 300 bar.

The Curcuma longa root extract can be commercially obtained from a number of providers, such as Parchem fine & specialty chemicals ("Curcuma longa"; CAS no. 84775-52-0), from Applied Food Sciences (as PurCL^{™}), or from Biogründl S.L. (ECURCD40OGE).

***Eugenia caryophyllus*** bud extract refers to the extract of cloves, which are the aromatic flower buds of a tree in the family Myrtaceae, *Syzygium aromaticum*, also known as *Eugenia caryophyllus.*

Cloves are used in the cuisine of Asian, African, and the Near and Middle East countries. Cloves are also employed in Indian Ayurvedic medicine, Chinese medicine, and western herbalism and dentistry where the essential oil is used as an anodyne for dental emergencies. Cloves are also known as carminatives to increase hydrochloric acid in the stomach and to improve peristalsis, and are additionally said to be a natural anthelmintic. Clove oil is used in aromatherapy when stimulation and warming are needed, especially for digestive problems.

The mixture of components extracted from *Eugenia caryophyllus* bud can comprise about 60-90% by weight eugenol, as well as acetyl eugenol, caryophyllene and other constituents such as calcium, potassium, magnesium, phosphorus, iron, manganese, folic acid, fatty acids (omega-3), vitamins C and K, or dietary fibre.

The *Eugenia caryophyllus* bud extract can be obtained by extracting the flower buds or a powder thereof with an organic solvent or an oil. In order to prepare the extract, the buds can be treated, in particular it can be boiled, cured, dried and/or brought to powder form before carrying out the extraction.

In an embodiment, the extraction is carried out with an organic solvent. Examples of suitable solvents are aromatic hydrocarbons, such as toluene or xylene; aliphatic hydrocarbons such as diethyl ether, heptane, pentane, hexane, cyclohexane; chlorinated hydrocarbons such as ethylene dichloride, dichloromethane, trichlorethylene; ketones such as acetone; esters such as ethyl acetate; alcohols such as methanol, ethanol, propanol or butanol, or glycerol or propylene glycol; or any mixture thereof. The organic solvent is preferably a solvent that is pharmaceutically acceptable, such as acetone, methanol, ethanol, propanol or butanol, glycerol, ethyl acetate, diethyl ether, hexane, cyclohexane or propylene glycol. After extraction, solids in the extraction mixture may be removed such as by filtration, and the solvent is optionally evaporated.

In another embodiment, the extraction is performed by adding at least one oil to the flower buds or the powder thereof, more specifically by macerating the flower buds with the oil. Preferably, the oil is an oil of natural origin. In a preferred embodiment, the oil is selected from soybean oil, sunflower oil, almond oil, safflower oil, corn oil, sesame oil, rapeseed oil, linseed oil, cottonseed oil, rice bran oil, perilla oil, castor oil, olive oil, evening primrose oil, tsubaki oil, coconut oil, palm oil, hemp seed oil, tung oil, kapok oil and tea seed oil. Preferably, the oil is a pharmaceutically acceptable oil. More preferably, the oil is sunflower oil. After extraction, solids in the extraction mixture may be removed such as by filtration.

In an embodiment, when an oil is employed for extracting the flower buds or the powder thereof, the final oil (the oil with the extracted components) comprises between about 20 and about 80%, preferably between about 20 and about 60%, more preferably between about 30 and about 50%, even more preferably about 40% by weight components extracted from the flower buds or powder thereof with respect to the total weight of the final oil. The final oil may additionally comprise added components such as at least one antioxidant selected from those described further below, which can be present in amounts of about 0.001% to about 3%, preferably about 0.1 to about 0.5%, more preferably about 0.3%, with respect to the total weight of the final oil. The remaining weight of the final oil (up to 100% weight) is the oil employed for the extraction.

In another embodiment the extraction is an extraction using supercritical carbon dioxide as the solvent For instance, such an extraction may be carried out with carbon dioxide at a temperature of carbon dioxide between about 31°C and about 70°C, preferably between about 24 and about 55°C, and at a pressure of carbon dioxide comprised between about 74 and about 300 bar, preferably between about 100 and about 300 bar.

*Eugenia caryophyllus* bud extract can be commercially obtained from a number of providers, such as Parchem fine & specialty chemicals ("Clove extract"; CAS no. 84961-50-2), Sigma-Aldrich ("Clove bud extract"; CAS no. 84961-50-2), or Biogründl S.L. (ECLAVD40OGE).

In an embodiment, the weight ratio of *Calendula officinalis* flower oil: *Curcuma longa* root extract:*Eugenia caryophyllus* bud extract in the combination is about 0.5-5 : 0.5-5 : 0.5-5. In a preferred embodiment, this ratio is about 0.5-2 : 0.5-2 : 0.5-2. More preferably, this ratio is about 0.5-1.5 : 0.5-1.5 : 0.5-1.5. In a preferred particular embodiment, the ratio is about 1.125:1:1. These ratios, as well as the ratios mentioned hereinafter involving *Calendula officinalis* flower oil, *Curcuma longa* root extract, and/or *Eugenia caryophyllus* bud extract are determined based on the weight of the extracts or oil comprising no additional (or the minimum amount possible of) solvent that may have been used during the extraction processes described above. In a preferred embodiment, these ratios are determined based on the weight of the extracts or oil as obtained after extraction with an oil as described above.

The weight is selected independently for each of these three ingredients (*Calendula officinalis* flower oil, *Curcuma longa* root extract, *Eugenia caryophyllus* bud extract).

In another aspect, the present invention is directed to a pharmaceutical composition comprising the combination of the invention as defined in any of the above described embodiments.

In addition to *Calendula officinalis* flower oil, *Curcuma longa* root extract, and *Eugenia caryophyllus* bud extract, which are herein understood as pharmaceutically active ingredients, the combination/composition of the invention may comprise further components such as pharmaceutically acceptable excipients.

In the context of the present invention, "pharmaceutically acceptable" means that the component characterised as pharmaceutically acceptable is tolerated physiologically, normally meaning that it is not toxic when it is used in a combination or pharmaceutical composition for the treatment of vulvodynia, and in particular it means that the component is suitable for application to the vulva or a part thereof without producing undue toxicity, irritation or allergic response.

The term "excipient" refers to components of a pharmaceutical composition other than an active ingredient. They preferably include a "carrier, adjuvant and/or vehicle". Carriers are forms to which substances are incorporated to improve the delivery and the effectiveness of active ingredients (Als). AI carriers are used in AI-delivery systems such as in controlled-release technology to prolong *in vivo* AI actions, decrease AI metabolism, and reduce AI delivery to the target sites of pharmacological actions. Adjuvant is a substance added to an AI product formulation that affects the action of the AI in a predictable way. Vehicle is an excipient or a substance, preferably without therapeutic action, used as a medium to give bulk for the administration of medicines. The selection of these excipients and the amounts to be used will depend on the form of application of the pharmaceutical composition.

Examples of an excipient are a film-forming agent, a binder, a bulking agent, a disintegrant, a lubricating agent, a diluent, an osmotic pressure regulator, a pH adjusting agent, a dispersant, an emulsifier, an antiseptic or disinfectant agent, a stabilizer, an emollient, an antioxidant, a colorant, a thickener, a fragrance, or a flavoring agent. In a preferred embodiment, the excipient is an emollient, an antioxidant, a fragrance or a mixture thereof.

Compositions in accordance with the invention include cosmetics, personal care products, cosmoceuticals and pharmaceutical preparations (medicaments).

The pharmaceutical compositions disclosed herein can be formulated for topical, oral, mucosal (e.g., nasal, pulmonary, sublingual, vaginal, buccal, or rectal), parenteral (e.g., subcutaneous, intravenous, bolus injection, intramuscular, or intraarterial), or transdermal administration to a patient. Preferably, the pharmaceutical compositions are formulated for topical administration, and more preferably they are formulated for topical administration to the vulva or to a part thereof. Thus the present invention advantageously provides the possibility to proceed whenever necessary or desirable, to very localized and selective "soft" treatments thanks to the topical application method.

Examples of pharmaceutical composition forms include, but are not limited to: tablets; caplets; capsules, such as soft elastic gelatin capsules; cachets; troches; lozenges; dispersions; suppositories; ointments; cataplasms (poultices); pastes; powders; dressings; creams; plasters; solutions; patches; aerosols (e.g., nasal sprays or inhalers); gels; liquid dosage forms suitable for oral or mucosal administration to a patient, including suspensions (e.g., aqueous or non-aqueous liquid suspensions, oil-in-water emulsions, or a water-in-oil liquid emulsions), solutions, and elixirs; liquid dosage forms suitable for parenteral administration to a patient; and sterile solids that can be reconstituted to provide liquid dosage forms suitable for parenteral administration to a patient.

Pharmaceutical compositions suitable for topical administration may be formulated as an oil, lotion, cream, serum, gel, stick, spray, ointment, liquid wash, soap bar, paste, foam, mousse, powder, wipe, patch, strip, hydrogel, aerosol and non-aerosol spray and similar forms. In a preferred embodiment, the pharmaceutical composition is in the form of an oil, cream or lotion. More preferably, it is in the form of an oil. Methods of formulating such compositions are well known in the art.

The pharmaceutical composition of the invention may have different structural and physical characteristics than its naturally occurring components. For example, in the case of pharmaceutical compositions suitable for topical administration such as oils, creams or lotions, the composition may adhere to the vulva or to a part thereof much longer than its natural components. Also the presence of excipients may make the composition have markedly different characteristics from the naturally occurring components. For instance, antioxidants allow improving the stability so that the composition spoils much more slowly than the naturally occurring components.

In an embodiment, the combination/composition further comprises at least one emollient. In the context of the present invention, the term "emollient" refers to substances that soften and make the skin smooth by preventing skin from losing moisture. In an embodiment, the weight ratio of the emollient or emollients to each or any of *Calendula officinalis* flower oil, *Curcuma longa* root extract or *Eugenia caryophyllus* bud extract is about 25-60:1, more preferably about 35-50:1.

Suitable emollients for use in the compositions disclosed herein include, but are not limited to an oil (preferably of natural origin), a silicone compound (e.g., dimethicone, cyclomethicone, dimethicone copolyol or a mixture of cyclopentasiloxane and dimethicone/vinyldimethicone cross polymer, cyclopentasiloxane polysilicone), polyols such as sorbitol, glycerin, propylene glycol, ethylene glycol, polyethylene glycol, caprylyl glycol, polypropylene glycol, 1,3-butane diol, hexylene glycol, isoprene glycol, xylitol; ethylhexyl palmitate; a triglyceride such as caprylic/capric triglyceride and fatty acid ester such as cetearyl isononanoate or cetyl palmitate.

In an embodiment, the emollient is an oil of natural origin selected from those described further above which is pharmaceutically acceptable. In a preferred embodiment, the natural oil is soybean, sunflower, olive or coconut oil, and more preferably it is sunflower oil, also known as Helianthus annuus seed oil, commercially available from a large number of sources, such as from Parchem fine & specialty chemicals ("Sunflower oil"; CAS no. 8001-21-6) or from Spectrum Chemical MFG Group ("Sunflower seed oil"; S1929).

In an embodiment, the weight ratio of the oil of natural origin (such as sunflower oil) to each or any of *Calendula officinalis* flower oil, *Curcuma longa* root extract or *Eugenia caryophyllus* bud extract is about 1-10:1, more preferably about 3-5:1.

In an embodiment, the emollient is Argania spinosa kernel oil, generally also known as Argan oil. Argan oil is extracted from the seeds of the berry of the argan tree (Argania spinosa L.), a plant species endemic to southwestern Morocco. Argan oil can be extracted from the argan tree berry kernels in different manners. In a preferred embodiment the kernels are extracted by grinding the kernels and pressing them, e.g. by cold-pressing, yielding pure, unrefined argan oil.

Argan oil has been known for decades for its moisturizing, nourishing and softening action of the skin, and hence is used in many preparations in dermatology for skin and hair. Argan oil contains in particular fatty acids, such as palmitic acid, stearic acid, oleic acid, or linoleic acid; sterols,; tocopherols; and vitamin E. Argan oil can be commercially obtained from a number of providers, such as from Charkit Chemical Company ("Argan oil"; CAS no. 223747-87-3); from Biomol GmbH ("Argania spinose kernel oil"; IAX-700-001), or from ieS LABO ("Argane deodorized oil"; HV116).

In an embodiment, the weight ratio of *Argania spinosa* kernel oil to each or any of *Calendula officinalis* flower oil, *Curcuma longa* root extract or *Eugenia caryophyllus* bud extract is about 25-60:1, more preferably about 35-45:1.

In a particularly preferred embodiment, the combination of the invention comprises at least two emollients, one of which is an oil of natural origin (e.g. sunflower oil) as described above, and the other is *Argania spinosa* kernel oil. In an embodiment, the ratios of the oil of natural origin and the *Argania spinosa* kernel oil to each or any of *Calendula officinalis* flower oil, *Curcuma longa* root extract or *Eugenia caryophyllus* bud extract are those described above.

In any of the above described embodiments, the combination/composition further comprises at least one antioxidant. The antioxidant is preferably selected from tocopherol and esters thereof such as tocopheryl acetate, tocopheryl linoleate or tocopheryl nicotinate; ascorbic acid and esters thereof such as ascorbyl palmitate or ascorbyl stearate; unsaturated fatty acids; 2,6-Di-tert-butyl-4-methylphenol (BHT); tert-butil-4-hidroxianisol (BHA); or 2-(1,1-Dimethylethyl)-1,4-benzenediol (TBHQ). More preferably, the antioxidant is preferably selected from BHT and tocopheryl acetate. In a further embodiment, the combination further comprises at least two antioxidants selected from the above list. Preferably the antioxidants are BHT and tocopheryl acetate.

In an embodiment, the weight ratio of total antioxidant to each or any of *Calendula officinalis* flower oil, *Curcuma longa* root extract or *Eugenia caryophyllus* bud extract is 1:10-20, preferably 1:14-18. The weight ratio corresponding to each individual antioxidant depends greatly on the type of antioxidant employed.

In a particular embodiment the combination/composition comprises the following components:

| |
|---|
| Calendula officinalis flower oil |
| Curcuma longa root extract |
| Eugenia caryophyllus bud extract |
| Helianthus annuus seed oil |
| Argania spinosa kernel oil |
| Tocopheryl acetate |
| BHT |

In a particular embodiment the combination/composition comprises the following components and weight ratios:

| | |
|---|---|
| Calendula officinalis flower oil | 1-3 |
| Curcuma longa root extract | 1-3 |
| Eugenia caryophyllus bud extract | 1-3 |
| Helianthus annuus seed oil | 1-15 |
| Argania spinosa kernel oil | 70-90 |
| Tocopheryl acetate | 0.01-0.05 |
| BHT | 0.05-0.15 |

In a particular embodiment, the combination/composition comprises the following components and weight ratios:

| | |
|---|---|
| Calendula officinalis flower oil | 2.25 |
| Curcuma longa root extract | 2.00 |
| Eugenia caryophyllus bud extract | 2.00 |
| Helianthus annuus seed oil | 8.72 |
| Argania spinosa kernel oil | 84.50 |
| Tocopheryl acetate | 0.03 |
| BHT | 0.1 |

In another embodiment, in any of the above embodiments, the combination/composition does not comprise, but consists of, the stated components.

In any of the above described embodiments, the combination/composition further comprises at least one fragrance. In the context of the present invention, a fragrance is an agent which makes the smell of the pharmaceutical composition more pleasant. Most fragrances are synthetically-made, and may mimic, amongst many others, the smell of lemon, honey, mint, sandalwood or lotus.

The weight is selected independently for each of these three ingredients (*Calendula officinalis* flower oil, *Curcuma longa* root extract, *Eugenia caryophyllus* bud extract). If further components such as pharmaceutically acceptable excipients are present in the pharmaceutical composition, the weight for each of the three ingredients is selected such that the sum of the weight of the three ingredients plus the weight of the further components does not exceed 100.0% with respect to the total weight of the pharmaceutical composition.

These weights, as well as the weights mentioned hereinafter involving *Calendula officinalis* flower oil, *Curcuma longa* root extract, and/or *Eugenia caryophyllus* bud extract, are determined based on the weight of the extracts or oil comprising no additional (or the minimum amount possible of) solvent or oil that may have been used during the extraction processes described above. In another embodiment, these weights are determined based on the weight of the extracts or oil as obtained after extraction with an oil as described above.

In an embodiment, the pharmaceutical composition further comprises at least one emollient as described above, in the following weight percentages with respect to the total weight of the pharmaceutical composition:
- Oil of natural origin: about 1-20%, preferably about 3-13%, more preferably about 8-10%; and/or
- Argania spinosa kernel oil: about 10-90%, preferably about 50-90%, more preferably about 80-90%.

In an embodiment, the pharmaceutical composition further comprises at least one antioxidant as described above. In an embodiment, the weight of antioxidant in the pharmaceutical composition is about 0.001% to about 3%, preferably about 0.01 to about 0.3%, with respect to the total weight of the pharmaceutical composition. In an embodiment, the pharmaceutical composition comprises the following antioxidants and weight percentages with respect to the total weight of the pharmaceutical composition:
- Tocopheryl acetate: about 0.1-0.001%, preferably about 0.05-0.01%; and/or
- BHT: about 1-0.01%, preferably about 0.5-0.05%.

In an embodiment, the pharmaceutical composition further comprises at least one fragrance as described above for the combination of the invention. In an embodiment, the weight of the fragrance is about 5-0.01%, preferably about 1 to 0.1%, with respect to the total weight of the pharmaceutical composition.

Additional excipients or materials may be also included in the pharmaceutical composition of the invention. The weight of the additional excipients or materials with respect to the total weight of the pharmaceutical composition will vary greatly depending on the type of excipient/material employed.

Any or all of the above described weight embodiments of the pharmaceutical composition may be combined with each other to arrive at new embodiments. It is to be understood that the weight percentages of each component are chosen such that the total percentage does not exceed 100% with respect to the total weight of the pharmaceutical composition. In an embodiment, the weight percentages of each component are chosen such that the total percentage does not reach 100% with respect to the total weight of the pharmaceutical composition, such that further components may be present. In an embodiment, the weight percentages of each component are chosen such that the total percentage totals 100% with respect to the total weight of the pharmaceutical composition, such that further components are not present, in which case the pharmaceutical composition consists of such components.

In a preferred particular embodiment, the combination/composition comprises or consists of the following components:

| |
|---|
| Argania spinosa kernel oil |
| Helianthus annuus seed oil |
| Calendula officinalis flower oil |
| Curcuma longa root extract |
| Eugenia caryophyllus bud extract |
| Tocopheryl acetate |
| Fragrance |
| BHT |

In a preferred particular embodiment, the combination/composition comprises or consists of the following components and weight ratios:

| | |
|---|---|
| Argania spinosa kernel oil | 70-90 |
| Helianthus annuus seed oil | 1-15 |
| Calendula officinalis flower oil | 1-3 |
| Curcuma longa root extract | 1-3 |
| Eugenia caryophyllus bud extract | 1-3 |
| Tocopheryl acetate | 0.01-0.05 |
| Fragrance | 0.1-1.0 |
| BHT | 0.05-0.15 |

In a preferred particular embodiment, the combination/composition presents the following composition and weight percentages:

| | |
|---|---|
| Argania spinosa kernel oil | 84.50 |
| Helianthus annuus seed oil | 8.72 |
| Calendula officinalis flower oil | 2.25 |
| Curcuma longa root extract | 2.00 |
| Eugenia caryophyllus bud extract | 2.00 |
| Tocopheryl acetate | 0.03 |
| Fragrance | 0.40 |
| BHT | 0.10 |

In another aspect, the present invention refers to a method for preparing the combination of the invention or the pharmaceutical composition of the invention.

The method of the invention comprises adding (to a vessel or reactor) the different above described components of the combination or pharmaceutical composition to provide a component mixture and homogenizing the mixture. In a preferred embodiment, each time that a component is added to another component or component mixture, homogenization is carried out after the addition, and if applicable, prior to addition of a further component. Preferably, the components are added one by one.

In a preferred embodiment, the following components are added in the following order:
- *Calendula officinalis* flower oil;
- *Eugenia caryophyllus* bud extract; and
- *Curcuma longa* root extract.

In an embodiment, the at least one emollient is added prior to the addition of *Calendula officinalis* flower oil, *Eugenia caryophyllus* bud extract, and *Curcuma longa* root extract.

In an embodiment, the at least one antioxidant is added after the addition of *Calendula officinalis* flower oil, *Eugenia caryophyllus* bud extract, and *Curcuma longa* root extract, and/or each of *Calendula officinalis* flower oil, *Eugenia caryophyllus* bud extract and *Curcuma longa* root extract can comprise the antioxidant as described above for each of the extracts.

In an embodiment, additional excipients are added after the addition of *Calendula officinalis* flower oil, *Eugenia caryophyllus* bud extract, and *Curcuma longa* root extract. It may be added prior to, at the same time as, or after the addition of the at least one antioxidant.

In an embodiment, addition of components is in the following order:
- Emollient;
- *Calendula officinalis* flower oil;
- *Eugenia caryophyllus* bud extract;
- *Curcuma longa* root extract;
- Antioxidant;
- Additional excipients such as fragrance.

In another embodiment, addition of components is in the following order:
- Emollient;
- *Calendula officinalis* flower oil comprising antioxidant;
- *Eugenia caryophyllus* bud extract comprising antioxidant;
- *Curcuma longa* root extract comprising antioxidant;
- Optionally antioxidant;
- Additional excipients such as fragrance.

In a preferred embodiment, the *Calendula officinalis* flower oil, *Eugenia caryophyllus* bud extract, and *Curcuma longa* root extract, and especially the *Eugenia caryophyllus* bud extract and *Curcuma longa* root extract, added are dissolved or dispersed in an oil of natural origin, in particular as obtained from the above described maceration processes.

In a preferred particular embodiment, addition of components is in the following order:
- Argania spinosa kernel oil;
- *Calendula officinalis* flower oil in sunflower oil comprising BHT;
- *Eugenia caryophyllus* bud extract in sunflower oil comprising tocopheryl acetate;
- *Curcuma longa* root extract in sunflower oil comprising tocopheryl acetate;
- BHT;
- Fragrance.

In a particular embodiment, the combination or pharmaceutical composition of the present invention does not comprise one or more of the following: rhubarb root extract, milk thistle extract, dandelion extract, feverfew extract, lemon extract or peppermint extract.

In a particular embodiment, the combination of pharmaceutical composition of the present invention does not comprise one or more of the following: Butylene glycol dicaprylate/dicaprate, octyldodecanol, diisostearyl malate, ethylhexyl methoxycinnamate, bis-ethylhexyloxyphenol methoxyphenyl triazine, ethylhexyl salicylate, octocrylene, silica silylate, polysilicone-15, dibutyl ethylhexanoyl glutamide, dibutyl lauroyl glutamide, butylene glycol, Morinda citrifolia leaf cell culture extract, Echinacea purpurea leaf/stem meristem cell culture extract, onsen-sui, sea water, Aloe barbadensis leaf extract, Cocos nucifera (coconut) oil, Gardenia tahitensis flower, tocopherol, Lavandula angustifolia (lavender) flower water, Angelica archangelica root extract, Viola tricolor extract, Achillea millefolium extract, Glycine soja (soybean) seed extract, Oryza sativa (rice) bran extract, Quillaja saponaria bark extract, Sapindus mukurossi fruit extract, Saponaria officinalis leaf extract, Sesamum indicum (sesame) seed extract, Paeonia suffruticosa root extract, algae extract, Alpinia officinarum root extract, Arnica montana flower extract, Artemisia vulgaris extract, Chrysanthemum indicum callus culture extract, Dicentra spectabilis callus culture extract, Dryopteris crassirhizoma extract, Garcinia mangostana fruit extract, Garcinia mangostana peel extract, Gentiana lutea root extract, Iris setosa callus culture extract, Terminalia chebula fruit extract, Undaria pinnatifida extract, Zinnia elegans callus culture extract, Atractyloides japonica rhizome extract, 1,2-hexanediol, phenoxyethanol, caprylyl glycol, disodium EDTA, ethylhexylglycerin, pentylene glycol, Houttuynia cordata extract, Paeonia albiflora root extract, Angelica keiskei extract, Centella asiatica extract, Perilla ocymoides leaf extract, Rosmarinus officinalis (rosemary) leaf extract, Taraxacum officinale (dandelion) rhizome/root extract, Diospyros kaki leaf extract, Cinnamomum cassia bark extract, Artemisia princeps leaf extract, Chrysanthellum indicum extract, Chaenomeles sinensis fruit extract, Camellia sinensis leaf extract, Cnidium officinale root extract, Citrus aurantium dulcis (orange) fruit extract, Musa sapientum (banana) fruit extract, Pyrus communis (pear) fruit extract, Pyrus malus (apple) fruit extract, Vitis vinifera (grape) fruit extract, glycerin, Melissa officinalis leaf extract, Alchemilla vulgaris extract, Ampelopsis japonica root extract, Angelica dahurica root extract, Ipomoea hederacea seed extract, Malva sylvestris (mallow) extract, Mentha piperita (peppermint) leaf extract, Morus alba root extract, Primula veris extract, silkworm extract, Veronica officinalis extract, Glycyrrhiza glabra (licorice) root extract, Poria cocos extract, Astragalus membranaceus root extract, Rehmannia chinensis root extract, Angelica acutiloba root extract, Asarum sieboldi root extract, Magnolia liliflora bud extract, Nardostachys chinensis root extract, Aspergillus ferment, Bambusa vulgaris extract, Bambusa vulgaris leaf/stem extract, Lysimachia foenum-graecum extract, Thymus vulgaris (thyme) extract, Vitex trifolia fruit extract, Allium sativum (garlic) bulb extract, Acanthopanax senticosus (eleuthero) root extract, Lycium chinense fruit extract, Portulaca oleracea extract, Scutellaria baicalensis root extract, Coix lacryma-jobi mayuen seed extract, Polygonum multiflorum root extract, Pueraria lobata root extract, Sophora angustifolia root extract, Zizyphus jujuba fruit extract, Angelica keiskei leaf/stem extract, Bletilla striata root extract, Castanea sativa (chestnut) leaf extract, Corn us officinal is fruit extract, Foeniculum vulgare (fennel) fruit extract, Ganoderma lucidum (mushroom) stem extract, Ginkgo biloba leaf extract, Juglans regia (walnut) seed extract, Pinus sylvestris bud extract, Plantago asiatica extract, Rubus idaeus (raspberry) fruit extract, Vigna radiata seed extract, Averrhoa carambola fruit extract, Caulerpa lentillifera extract, Codium fragile extract, Corchorus olitorius leaf extract, Epilobium angustifolium extract, Harpagophytum procumbens root extract, Lepidium meyenii root extract, Moringa oleifera leaf extract, Paullinia cupana seed extract, Usnea barbata (lichen) extract, Zanthoxylum piperitum fruit extract, Saccharomyces!Viscum album (mistletoe) ferment extract, Saccharomyces/lmperata cylindrica root ferment extract, Lactobacillus/soybean ferment extract, Soleirolia soleirolii extract, Origanum majorana leaf extract, Glycine soja (soybean) seed extract, Oryza sativa (rice) extract, Phyllostachys nigra extract, Rubus fruticosus (blackberry) fruit extract, Sesamum indicum (sesame) seed extract, Nelumbo nucifera flower extract, Achyranthes fauriei root extract, Adenophora stricta root extract, Agrimonia eupatoria extract, Akebia qui nata stem extract, Angelica gigas root extract, Angelica tenuissima root extract, Arctium lappa root extract, Areca catechu seed extract, Artemisia absinthium extract, Asparagus cochinchinensis root extract, Atractyloides chinensis rhizome extract, Belamcanda chinensis root extract, Betula alba bark extract, Buddleja officinalis leaf extract, Carthamus tinctorius (safflower) flower extract, Chrysanthemum morifolium flower extract, Cimicifuga simplex root extract, Citrus unshiu peel extract, Corydalis turtschaninovii root extract, Cyperus rotundus root extract, Daemonorops draco extract, Dioscorea japonica root extract, Dryopteris filix-mas root extract, Epimedium grandiflorum flower/leaf/stem extract, Forsythia suspensa fruit extract, Ganoderma lucidum (mushroom) extract, Gastrodia elata root extract, Inonotus obliquus (mushroom) extract, Inula helenium extract, Lilium tigrinum extract, Lindera strychnifolia root extract, Magnolia obovate bark extract, Morus alba bark extract, Oenothera biennis (evening primrose) seed extract, Oldenlandia diffusa extract, Ophiopogon japonicus root extract, Panax notoginseng root extract, Perilla ocymoides seed extract, Plantago ovata seed extract, Pogostemon cablin leaf extract, Polygala tenuifolia root extract, Pterocarpus santalinus wood extract, Rhodiola sacra root extract, Rubia cordifolia root extract, Rubus chingii fruit extract, Rumex crispus root extract, Saururus chinensis extract, Schizandra chinensis fruit extract, Scrophularia buergeriana root extract, Taraxacum officinale (dandelion) leaf extract, Trichosanthes kirilowii root extract, velvet extract, Viscum album (mistletoe) extract, Zanthoxylum alatum fruit extract, Ribes nigrum (black currant) leaf extract, Bupleurum chinense root extract, Codonopsis lanceolate root extract, Commiphora myrrha resin extract, Cordyceps sinensis extract, Eclipta prostrata extract, Eucommia ulmoides leaf extract, Lonicera japonica (honeysuckle) flower extract, Myristica fragrans (nutmeg) extract, Pinellia ternata root extract, Platycodon grandiflorum root extract, Poncirus trifoliata fruit extract, Prunus persica (peach) kernel extract, Salvia miltiorrhiza root extract, Sophora japonica bud extract, Ulmus davidiana root extract, Oenothera biennis (evening primrose) flower extract, Prunus persica (peach) flower extract, Saccharomyces/Panax ginseng flower ferment extract, or Sigesbeckia orientalis extract.

In a particular embodiment, the combination of pharmaceutical composition of the present invention does not comprise Butylene glycol dicaprylate/dicaprate.

In a particular embodiment, the combination of pharmaceutical composition of the present invention does not comprise octyldodecanol.

In a particular embodiment, the combination of pharmaceutical composition of the present invention does not comprise diisostearyl malate.

In a particular embodiment, the combination of pharmaceutical composition of the present invention does not comprise ethylhexyl methoxycinnamate.

In a particular embodiment, the combination of pharmaceutical composition of the present invention does not comprise bis-ethylhexyloxyphenol methoxyphenyl triazine.

In a particular embodiment, the combination of pharmaceutical composition of the present invention does not comprise ethylhexyl salicylate.

In a particular embodiment, the combination of pharmaceutical composition of the present invention does not comprise octocrylene.

In another aspect, the invention refers to the combination of the invention, or to the pharmaceutical composition of the invention, for use in medicine, and more specifically for use in the treatment and/or prevention of vulvodynia.

Similarly, it is also disclosed but not part of the invention the use of the combination of the invention, or of the pharmaceutical composition of the invention, in the manufacture of a medicament for the treatment and/or prevention of vulvodynia.

Similarly, the invention refers to the use of the combination of the invention, or of the pharmaceutical composition of the invention, for treating and/or preventing vulvodynia.

Similarly, it is also disclosed but not part of the invention, a method of treating vulvodynia comprising administering to a subject in need thereof the combination of the invention, or of the pharmaceutical composition of the invention.

It is to be understood that any use of the combinations or pharmaceutical compositions herein described comprise said combinations or pharmaceutical compositions in a therapeutically effective amount. The physician will determine the amount (dosage) which is therapeutically effective according to the type of pharmaceutical formulation or form of administration chosen. Pharmaceutical formulations and modes of administration are described hereinabove. Other factors that will influence the chosen amount (dosage) are the age of the patient, and the type and severity of the vuvlodynia being treated. In an embodiment, *Calendula officinalis* flower oil, *Curcuma longa* root extract, and *Eugenia caryophyllus* bud extract are used in a therapeutically effective amount.

Vulvodynia is a recognised disorder referred to in the World Health Organization (WHO) ICD-10 classification under code N94.81. The classification describes the disease as follows: "*Complex pain syndrome with unknown etiology, characterized by constant or intermittent generalized vulva pain (generalized vulvodynia) or localized burning sensations in the vestibule area when pressure is applied (vestibulodynia, or vulvar vestibulitis syndrome). Typically, vulvar tissue with vulvodynia appears normal without infection or skin disease. Vulvodynia impacts negatively on a woman's quality of life* as *it interferes with sexual and daily activities".*

Thus, in an embodiment, the vulvodynia is generalized vulvodynia. Generalised vulvodynia affects the vulva as a whole.

In another embodiment, the vulvodynia is localized vulvodynia. Localized vulvodynia affects at least a portion of the vulvar vestibule or the clitoris. In the former case, the vulvodynia is referred to as vestibulodynia, or vulvar vestibulitis syndrome. In the latter case, the vulvodynia is referred to as clitorodynia.

In line with the above WHO definition, representatives from the International Society for the Study of Vulvovaginal Disease, the International Society for the Study of Women's Sexual Health, and the International Pelvic Pain Society have developed consensus terminology and classified vuvlodynia as persistent vulvar pain without an identifiable etiology and of at least three months duration. Thus, in an embodiment, vulvodynia involves vulvar pain for at least three months.

As used herein, the term "treatment" or derivations thereof include the eradication, removal, reversion, alleviation, modification, or control of the vulvodynia. In an embodiment, the invention is also directed to the prevention of vulvodynia. The term "prevention" or derivations thereof refer to the avoiding or minimizing of the onset or recurrence of the vulvodynia.

The subject which is treated is a female subject, either human or animal, preferably a female human subject.

In an embodiment, the treatment of vulvodynia involves the administration of the combination or pharmaceutical composition of the invention once daily, twice daily, or thrice daily. Preferably administration is twice daily. The treatment is ideally maintained until the vulvodynia is reversed and controlled. In an embodiment, the treatment is performed during at least one month, at least three months, or at least half a year.

Once the vulvodynia is reversed and controlled, the combination or pharmaceutical composition of the invention may be administered in a preventive manner with the same frequency, or in a lesser frequency, such as once, twice, or thrice per week.

In an embodiment, the treatment of vulvodynia involves further administering a further pharmaceutically active ingredient selected from those described hereinbelow. The present invention is hence also directed in a further aspect to a combination or pharmaceutical composition of the invention further comprising a further pharmaceutically active ingredient selected from those described hereinbelow.

The further pharmaceutically active ingredient may be provided as part of the combination or pharmaceutical composition of the invention, or as a separate pharmaceutical composition, in particular for administration at the same time or at different times. When administered separately and at different times, either the combination/pharmaceutical composition of the invention, or the separate pharmaceutical composition, may be administered first. In addition, both pharmaceutical compositions can be administered in the same day or at different days, and they can be administered using the same schedule or at different schedules during the treatment cycle. In an embodiment, the pharmaceutical composition of the invention and the separate pharmaceutical composition are the same type of pharmaceutical composition form. In another embodiment, the pharmaceutical composition of the invention and the separate pharmaceutical composition are a different type of pharmaceutical composition form. The type of pharmaceutical composition form of the separate pharmaceutical composition is also selected from the hereinabove described list of forms of pharmaceutical composition.

In an embodiment, the further pharmaceutically active ingredient is selected from sodium channel blockers, such as lidocaine, benzocaine, pramoxine, doxepin, benzydamine, dyclonine, bupivacaine, prilocaine, tetracaine, procaine, cinchocaine, amitriptyline, doxepin, GTX2,3 and GTX1,4, neosaxitoxin and tetrodoxin; corticosteroids, in particular local corticosteroids, such as cortisol, corticosterone, cortisone or aldosterone; mast cell stabilizers such as nedocromil or cromolyn; non-pungent TRPV-1 agonists such as N-palmitoyl-vanillamide, arvanil, olvanil; endogenous cannabinoids and cannabinoids such as palmitoylethanolamide (PEA), anandamide and cannabidiol; gabapentinoids such as gabapentin or pregabalin; potassium channel openers and modulators such as diclofenac, retigabine, flupirtine or cromakalim or baclofen; alpha-2 adrenergic agonists such as clonidine or dexmedetomidine, or NMDA antagonists such as ketamine; or combinations thereof. In a preferred embodiment, the further pharmaceutically active ingredient is selected from sodium channel blockers, preferably lidocaine; and corticosteroids.

In an embodiment, the medical uses herein described are complemented with at least one further therapy. In an embodiment, the further therapy is selected from psychological therapy, including cognitive behavioral therapy; physical therapy, such as for improving pelvic floor tone, or massaging vulva; oral agent administration (non-pharmaceutical), such as administration of an oil of natural origin, in particular selected from those described hereinabove, such as evening primrose oil; or surgical intervention, such as vestibulectomy. In a particular embodiment, the further therapy is selected from physical therapy, such as massaging vulva; and/or the administration of the oil of natural origin, in particular selected from those described hereinabove, such as evening primrose oil. This oil may be administered similarly to the further pharmaceutical ingredient, e.g. it may be administered in a form selected from those described hereinabove for the pharmaceutical compositions of the invention, such as in a form suitable for oral administration, e.g. in the form of a capsule. In a more particular embodiment, the combination or pharmaceutical composition of the invention is administered in a form suitable for topical application, e.g. as an oil, and the oil of natural origin is administered in a form suitable for oral administration, e.g. in the form of a capsule or a tablet. The oil of natural origin in a form suitable for oral administration can be obtained from different commercial sources, such as from HTC Health (as "Evening Primrose Oil - Vegetarian soft shell") or Livealth BioPharma Pvt. Ltd. (as "Livprime - 1000"). The oil of natural origin in a form suitable for oral administration can be administered for instance four times a day, e.g. twice every 12 hour interval.

In an embodiment, when any or all of the combination or pharmaceutical composition of the invention, or the further pharmaceutically active ingredient or separate pharmaceutical composition comprising it, or the further therapy involve topical application, the topical application is carried out by placing an amount of the relevant topical agent on the hand and then spreading the agent on the surface of the skin of the vulvar area where pain radiates.

As used herein, the term "about" means a slight variation of the value specified, preferably within 10 percent of the value specified. Nevertheless, the term "about" can mean a higher tolerance of variation depending on for instance the experimental technique used. Said variations of a specified value are understood by the skilled person and are within the context of the present invention. Further, to provide a more concise description, some of the quantitative expressions given herein are not qualified with the term "about". It is understood that, whether the term "about" is used explicitly or not, every quantity given herein is meant to refer to the actual given value, and it is also meant to refer to the approximation to such given value that would reasonably be inferred based on the ordinary skill in the art, including equivalents and approximations due to the experimental and/or measurement conditions for such given value.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "about 1 % to about 5 %" should be interpreted to include not only the explicitly recited values of about 1 % to about 5 %, but also include individual values and sub-ranges within the indicated range. Thus, included in this numerical range are individual values such as 2, 3. and 4 and sub-ranges such as from 1-3, from 2-4, and from 3-5, etc. This same principle applies to ranges reciting only one numerical value.

The following examples are merely illustrative of certain embodiments of the invention and cannot be considered as restricting it in any way.

### Examples

### Example 1: Preparation of a pharmaceutical composition of the invention

The Pharmaceutical Composition of the invention was prepared by adding the following components in the following order
- Argania spinosa kernel oil was added to a reactor;
- *Calendula officinalis* flower oil in sunflower oil comprising BHT (Provital S.A.; 1210000G) was then added, and the resulting mixture was homogenized;
- *Eugenia caryophyllus* bud extract in sunflower oil comprising tocopheryl acetate (Biogründl S.L.; ECLAVD40OGE) was then added, and the resulting mixture was homogenized;
- *Curcuma longa* root extract in sunflower oil comprising tocopheryl acetate (Biogründl S.L.; ECURCD40OGE) was then added, and the resulting mixture was homogenized;
- BHT was then added, and the resulting mixture was homogenized;
- Lotus perfume (frangrance) was then added, and the resulting mixture was homogenized.

The above procedure yielded the following composition:

| Component | % wt. wrt composition wt. |
|---|---|
| Argania spinosa kernel oil | 84.50 |
| Helianthus annuus seed oil | 8.72 |
| Calendula officinalis flower oil | 2.25 |
| Curcuma longa root extract | 2.00 |
| Eugenia caryophyllus bud extract | 2.00 |
| Tocopheryl acetate | 0.03 |
| Fragrance | 0.40 |
| BHT | 0.10 |

### Example 2: Treatment of vulvodynia with a pharmaceutical composition of the invention (clinical case)

A 43-year-old patient who is distressed because for more than 6 months she has experienced burning and pain located in the vulvar area, not always caused by sexual contact, but pain prevents her from having sexual intercourse.

Patient is remitted by her dermatologist who has treated her with different topical treatments without positive results.

The relevant data of the clinical case is that the patient noticed discomfort and pain in the vulvar area at rubbing, but above all, what worried her the most was the pain she felt when having sex, especially with coital penetration.

Patient history is the following:
- Menarche at 14 years, with regular menstrual cycles
- Parity: 2002.
- Eutocic births at 33 and 37 years
- Contraception: Condoms
- No surgical interventions
- Important diseases: Family history of breast cancer, mother at age 50 and grandmother at age 65
- Congenital hiatus hernia
- Regarding toxic habits: very occasional alcohol consumer.
- BMI: 26.86
- Repetitive vulvovaginal infections by *candida albicans*: In the last two years, patient has presented 5 episodes that have required treatment with local antifungals, in ovum and in cream form.
- Prior pharmacological treatments (arrested before treatment with composition of the invention):
   ∘ Exomeprazole;
   ∘ Local corticosteroids; and
   ∘ Ocasionally, lidocaine pain cream.

Gynecological inspection was carried out and the following was observed:
- Vulvar mucosa without visible lesions, except a very mild erythematous area on the posterior vulvar hairpin.
- Pain on palpation in the vulvar vestibule with a swab and on introduction of the speculum, trigger points at 5 o'clock and 7 o'clock.
- Vagina without visible lesions, no leukorrhea.
- AVF uterus, no palpable attachments, mobile cervix, not pain upon mobilization
- The result of cervico-vaginal cytology is reported as normal
- Transvaginal ultrasound refers internal genitalia

Medical conclusion: The inspection of the vulva leads to a diagnosis of vulvodynia, specifically vestibulodynia.

### 1^{st} medical visit

The patient is informed about the probable diagnosis, as well as the possible triggers and sustainers of the pathology. Explaining of repercussions at a psychological and relational level. Women with vulvodynia and sexual pain commonly exhibit myofascial trigger points and increased muscle tension in the pelvis, abdomen, back and pelvic floor muscles.

The following prescriptions are made to reduce pain: application of pharmaceutical composition of the invention in oil form in the entire vulvar area and vaginal introitus in the morning and at night. Evening primrose oil (2 tablets / 12h) is recommended. Physiotherapy of the pelvic floor is recommended, self-administered massage in the vaginal introitus once a day with the pharmaceutical composition of the invention. Avoidance of synthetic underwear and sanitary napkins is recommended.

The next visit is scheduled in 1 months' time.

### 2^{nd} medical visit (at 1 month of treatment)

The patient reports improvement, as the vulvar pain has practically been reduced to sexual contact and in the area of the introitus, in the trigger points, and the pain is of less intensity than at the beginning of the treatment.

Patient evaluates in a very positive way the daily application of the vulvar oil, highlighting comfort sensation that it provides, the disappearance of the sensation of burning approximately from the 15^{th} day of treatment. Controlled sexual relationships are now possible.

Genital examination shows a more normalized tone of the pelvic floor muscles, less pain on palpation, better hydration of the vulvar area, and less sensitivity in the trigger points.

Patient is advised to continue treatment and next visit is scheduled in two months' time.

### 3^{rd} medical visit (at 3 months of treatment)

The patient reports feeling much better and has normalized her sexual activity. The pain is practically non-existent, except for occasional tolerable burning sensation.

In the genital examination, no visible alterations in the vulva and vagina are observed, muscle tone in the pelvic floor is adequate, trigger points practically non-existent.

Medical conclusion: the vulvodynia is reversed and controlled.

## Claims

1. Combination comprising:
- *Calendula officinalis* flower oil;
- *Curcuma longa* root extract; and
- *Eugenia caryophyllus* bud extract,
each present in an amount of 1-3% by weight with respect to the total weight of the combination.

2. Pharmaceutical composition comprising:
- *Calendula officinalis* flower oil;
- *Curcuma longa* root extract; and
- *Eugenia caryophyllus* bud extract,
each present in an amount of 1-3% by weight with respect to the total weight of the pharmaceutical composition,
and at least one pharmaceutically acceptable excipient.

3. Pharmaceutical composition according to claim 2, comprising at least one emollient.

4. Pharmaceutical composition according to claim 3, wherein the emollient is *Argania spinosa* kernel oil.

5. Pharmaceutical composition according to any one of claims 2 to 4, comprising at least one antioxidant.

6. Pharmaceutical composition according to claim 5, wherein the antioxidant is tocopherol or an ester thereof; an unsaturated fatty acid; 2,6-Di-tert-butyl-4-methylphenol (BHT); tert-butil-4-hidroxianisol (BHA); 2-(1,1-Dimethylethyl)-1,4-benzenediol (TBHQ); or a combination thereof.

7. Pharmaceutical composition according to any one of claims 2 to 6, further comprising a pharmaceutically active ingredient selected from sodium channel blockers; corticosteroids; mast cell stabilizers; endogenous cannabinoids and cannabinoids; gabapentinoids; potassium channel openers and modulators; alpha-2 adrenergic agonists; and NMDA antagonists.

8. Pharmaceutical composition according to any one of claims 2 to 7, wherein the *Calendula officinalis* flower oil; *Curcuma longa* root extract; and/or *Eugenia caryophyllus* are dissolved or dispersed in an oil of natural origin.

9. Pharmaceutical composition according to any one of claims 2 to 8, in a form suitable for topical application.

10. Method for the preparation of a pharmaceutical composition as defined in any one of claims 2 to 9, comprising the sequence of steps of:
- Adding a first component of the pharmaceutical composition to a vessel or reactor;
- Adding the remainder of components of the pharmaceutical composition one by one to the vessel or reactor, and homogenizing the mixture resulting after each addition of a component before addition of the next component.

11. Pharmaceutical composition as defined in any one of claims 2 to 9, for use in medicine.

12. Pharmaceutical composition as defined in any one of claims 2 to 9 for use in the treatment and/or prevention of vulvodynia.

13. Pharmaceutical composition for use according to claim 12, wherein the vulvodynia is generalized vulvodynia.

14. Pharmaceutical composition for use according to claim 12, wherein the vulvodynia is localized vulvodynia.

## Patentansprüche

1. Kombination bestehend aus:
- *Calendula officinalis* Blutenöl;
- *Curcuma longa* Wurzelextrakt; und
- *Eugenia caryophyllus* Knospenextrakt,
jeweils vorhanden in einer Menge von 1 bis 3 Gew.- %, bezogen auf das Gesamtgewicht der Kombination.

2. Pharmazeutische Zusammensetzung umfassend:
- *Calendula officinalis* Blütenöl;
- *Curcuma longa* Wurzelextrakt; und
- *Eugenia caryophyllus* Knospenextrakt,
jeweils vorhanden in einer Menge von 1 bis 3 Gew.- %, bezogen auf das Gesamtgewicht der pharmazeutischen Zusammensetzung,
und mindestens einen pharmazeutisch akzeptablen Hilfsstoff.

3. Pharmazeutische Zusammensetzung nach Anspruch 2, umfassend mindestens ein Emolliens.

4. Pharmazeutische Zusammensetzung nach Anspruch 3, wobei das Emolliens *Argania spinosa* Kernöl ist.

5. Pharmazeutische Zusammensetzung nach einem der Ansprüche 2 bis 4, umfassend mindestens ein Antioxidans.

6. Pharmazeutische Zusammensetzung nach Anspruch 5, wobei das Antioxidans Tocopherol oder ein Ester davon; eine ungesättigte Fettsäure; 2,6-Di-tert-butyl-4-methylphenol (BHT); tert-Butil-4-hidroxianisol (BHA); 2-(1,1-Dimethylethyl)-1,4-benzoldiol (TBHQ); oder eine Kombination davon ist.

7. Pharmazeutische Zusammensetzung nach einem der Ansprüche 2 bis 6, ferner umfassend einen pharmazeutischen Wirkstoff, ausgewählt aus Natriumkanalblockern; Kortikosteroiden; Mastzellstabilisatoren; endogenen Cannabinoiden und Cannabinoiden; Gabapentinoiden; Kaliumkanalöffnern und -modulatoren; alpha-2-adrenergen Agonisten; und NMDA-Antagonisten.

8. Pharmazeutische Zusammensetzung nach einem der Ansprüche 2 bis 7, wobei das *Calendula officinalis* Blütenöl; der *Curcuma longa* Wurzelextrakt; und/oder *Eugenia caryophyllus* in einem Öl natürlichen Ursprungs gelöst oder dispergiert sind.

9. Pharmazeutische Zusammensetzung nach einem der Ansprüche 2 bis 8 in einer für die topische Anwendung geeigneten Form.

10. Verfahren zur Herstellung einer pharmazeutischen Zusammensetzung wie in einem der Ansprüche 2 bis 9 definiert, umfassend die Abfolge der folgenden Schritte:
- Hinzufügen einer ersten Komponente der pharmazeutischen Zusammensetzung zu einem Behälter oder Reaktor;
- Zugabe des Rests der Komponenten der pharmazeutischen Zusammensetzung nacheinander in das Gefäß oder den Reaktor und Homogenisieren der nach jeder Zugabe einer Komponente resultierenden Mischung vor der Zugabe der nächsten Komponente.

11. Pharmazeutische Zusammensetzung wie in einem der Ansprüche 2 bis 9 definiert, zur Verwendung in der Medizin.

12. Pharmazeutische Zusammensetzung wie in einem der Ansprüche 2 bis 9 definiert zur Verwendung bei der Behandlung und/oder Prävention von Vulvodynie.

13. Pharmazeutische Zusammensetzung zur Verwendung nach Anspruch 12, wobei die Vulvodynie generalisierte Vulvodynie ist.

14. Pharmazeutische Zusammensetzung zur Verwendung nach Anspruch 12, wobei die Vulvodynie lokalisierte Vulvodynie ist.

## Revendications

1. Une combinaison comprenant :
- huile de fleur de *Calendula officinalis* ;
- extrait de racine de *Curcuma longa* ; et
- extrait de bourgeon d'*Eugenia caryophyllus*,
chacun présent en une quantité de 1 à 3 % en poids par rapport au poids total de la combinaison.

2. Composition pharmaceutique comprenant :
- huile de fleur de *Calendula officinalis* ;
- extrait de racine de *Curcuma longa* ; et
- extrait de bourgeon d'*Eugenia caryophyllus*,
chacun présent à raison de 1 à 3 % en poids par rapport au poids total de la composition pharmaceutique,
et au moins un excipient pharmaceutiquement acceptable.

3. Composition pharmaceutique selon la revendication 2, comprenant au moins un émollient.

4. Composition pharmaceutique selon la revendication 3, dans laquelle l'émollient est l'huile de noyau d'*Argania spinosa.*

5. Composition pharmaceutique selon l'une quelconque des revendications 2 à 4, comprenant au moins un antioxydant.

6. Composition pharmaceutique selon la revendication 5, dans laquelle l'antioxydant est le tocophérol ou un ester de celui-ci ; un acide gras insaturé ; le 2,6-Di-tert-butyl-4-méthylphénol (BHT) ; le tert-butil-4-hidroxianisol (BHA) ; le 2-(1,1-Diméthyléthyl)-1,4-benzènediol (TBHQ) ; ou une combinaison de ceux-ci.

7. Composition pharmaceutique selon l'une quelconque des revendications 2 à 6, comprenant en outre un ingrédient pharmaceutiquement actif choisi parmi les bloqueurs de canaux sodiques ; les corticostéroïdes ; les stabilisateurs de mastocytes ; les cannabinoïdes et cannabinoïdes endogènes ; les gabapentinoïdes ; les ouvreurs et modulateurs de canaux potassiques ; les agonistes alpha-2 adrénergiques ; et les antagonistes de NMDA.

8. Composition pharmaceutique selon l'une quelconque des revendications 2 à 7, dans laquelle l'huile de fleur de *Calendula officinalis* ; l'extrait de racine de *Curcuma longa* ; et/ou *Eugenia caryophyllus* sont dissous ou dispersés dans une huile d'origine naturelle.

9. Composition pharmaceutique selon l'une quelconque des revendications 2 à 8, sous une forme appropriée pour une application topique.

10. Procédé de préparation d'une composition pharmaceutique telle que définie dans l'une quelconque des revendications 2 à 9, comprenant la séquence d'étapes consistant à :
- Ajouter un premier composant de la composition pharmaceutique dans une cuve ou un réacteur ;
- Ajouter le reste des composants de la composition pharmaceutique un par un dans la cuve ou le réacteur, et homogénéiser le mélange résultant après chaque ajout d'un composant avant l'ajout du composant suivant.

11. Composition pharmaceutique telle que définie dans l'une quelconque des revendications 2 à 9, pour une utilisation en médecine.

12. Composition pharmaceutique telle que définie dans l'une quelconque des revendications 2 à 9 pour une utilisation dans le traitement et/ou la prévention de la vulvodynie.

13. Composition pharmaceutique pour l'utilisation selon la revendication 12, dans laquelle la vulvodynie est une vulvodynie généralisée.

14. Composition pharmaceutique pour l'utilisation selon la revendication 12, dans laquelle la vulvodynie est une vulvodynie localisée.
